(19) **Europäisches Patentamt**
**European Patent Office**
**Office européen des brevets**

(11) **EP 4 570 353 A1**

(12) **EUROPEAN PATENT APPLICATION**

(43) Date of publication:
**18.06.2025 Bulletin 2025/25**

(21) Application number: **24213868.3**

(22) Date of filing: **19.11.2024**

(51) International Patent Classification (IPC):
**B01D 53/00** (2006.01)  **B01D 53/04** (2006.01)
**B01D 53/30** (2006.01)  **C07C 7/12** (2006.01)
**C07C 11/24** (2006.01)

(52) Cooperative Patent Classification (CPC):
**B01D 53/002; B01D 53/04; B01D 53/30;**
**C07C 7/12; C07C 11/24;** B01D 2256/24;
B01D 2257/702; B01D 2258/025; B01D 2259/4525

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(30) Priority: **08.12.2023 JP 2023207639**

(71) Applicant: **L'AIR LIQUIDE, SOCIETE ANONYME**
**POUR L'ETUDE ET**
**L'EXPLOITATION DES PROCEDES GEORGES**
**CLAUDE**
**75007 Paris (FR)**

(72) Inventors:
• **LAVENN, Christophe**
**78350 Jouy-en-Josas (FR)**
• **BENZAQUI, Marvin**
**78350 Jouy-en-Josas (FR)**
• **OGAWA, Tomofumi**
**Kanagawa, 239-0847 (JP)**
• **PROST, Laurent**
**60439 Frankfurt am Main (DE)**

(74) Representative: **Air Liquide**
**L'Air Liquide S.A.**
**Direction de la Propriété Intellectuelle**
**75, Quai d'Orsay**
**75321 Paris Cedex 07 (FR)**

(54) **GAS REFINING SYSTEM FOR REMOVING CONTAMINANTS IN GAS**

(57)  [Problem] To provide a gas refining system for removing trace impurities in gas.

[Solution] A gas refining system 1 comprises: a system unit 11 comprising a gas source unit 111 that comprises a storage container, a purifier unit 113 for removing residual solvent from the gas, and a refining unit 112 for controlling the purification operations of the purifier unit 113; an evaluation unit 13 for assessing the status of at least the purifier unit 113 by means of rule-based evaluation conditions and/or a machine learning model 131 based on one or more kinds of data from among system parameters 1100, system monitoring data 1200, and user parameters 1300; and a control unit 15 comprising a purifier control unit 152 for controlling the purifier unit 113 based on data assessed by the evaluation unit 13.

【Figure 1】

EP 4 570 353 A1

**Description**

[Technical Field]

The present disclosure relates to a gas refining system for removing contaminants in gas. More specifically, the present disclosure relates to a gas refining system for removing trace impurities in gas (smart refining system).

[Background Art]

In low-pressure/vacuum carburizing techniques, heat treatment at low pressure/vacuum is used to modify the surface of metal components produced from iron, steel, and other metals and alloys. Low-pressure/vacuum carburization also generates less soot compared with other carburizing methods (for example, the high-concentration carburizing method and the gas carburizing method), and is therefore attracting attention (for example, see Patent Document 1).

In carburizing techniques, however, acetylene ($C_2H_2$) for example is most widely used as the carbon source (for example, see Patent Document 2).

In low-pressure/vacuum carburizing processes, acetylene must be introduced into a chamber at a constant flow rate. However, in contrast to other hydrocarbon gases, acetylene cannot be safely compressed at a pressure higher than 1.5 bar. Acetylene dissolved in an organic solvent (such as acetone or dimethylformamide (DMF)) is therefore impregnated into a porous material (such as a porous calcium silicate material) as a way to store acetylene.

When the acetylene in the organic solvent is recovered from the porous material, some organic solvent inevitably remains in the acetylene gas.

In carburizing processes, a plurality of storage containers (such as cylinders) storing acetylene are installed, and the amount of acetylene supplied to a chamber must be controlled in accordance with processing requirements.

Organic solvent (referred to as "residual solvent") is contained to some extent in the acetylene gas released from the storage container. The concentration of organic solvent in the acetylene gas depends on the nature of the solvent, the temperature, the residual pressure in the cylinder, and the rate at which the acetylene gas is taken out (see Patent Document 3, for example).

When the organic solvent is dimethylformamide (DMF), for example, the concentration of the organic solvent in the acetylene gas is in the range of about 0.01% (100 ppm) to about 1% (10,000 ppm). When the organic solvent is acetone, the content ranges from about 1% to about 10%.

The presence of organic solvent during the carburizing process may adversely affect process homogeneity and quality, and may also affect maintenance, etc. The concentration of the organic solvent in the acetylene must therefore be controlled so as not to exceed a prescribed concentration.

Meanwhile, an organic solvent is needed to store the acetylene in storage containers, but the organic gas is also carried along with the acetylene in order to extract the acetylene gas from the storage containers, and it is furthermore difficult to extract all of the acetylene from the storage containers.

The dimethylformamide (DMF) content in acetylene gas is also about 10 to 100 times lower than that of acetone. Dimethylformamide (DMF) is therefore preferably used rather than acetone (see Patent Document 4, for example).

In some regions, however, the use of DMF is restricted by laws, regulations, and safety guidelines, for example. The use of acetone as the organic solvent is therefore sometimes recommended.

Examples of methods for removing residual solvent from acetylene gas include cold traps (for example, Patent Document 5) or solvent removal devices comprising adsorbents (for example, see Patent Documents 3 and 6). Such solvent removal devices (also referred to as purifiers) must be strictly monitored, and the flow rate at which the acetylene gas is introduced must be limited. The content of the organic solvent in acetylene may vary over time or during use. Specifically, the size, installation conditions, and operating conditions, for example, of solvent removal devices (purifiers) are difficult to establish. Thus, when acetylene gas (containing organic solvent) is used for an application such as a carburizing process, the solvent removal device (purifier) must be of an appropriate size and must be maintained and controlled (i.e., installation conditions and operating conditions).

When acetylene gas is taken out of a storage container (such as a cylinder), the residual pressure inside the storage container gradually decreases, which means that the acetylene content inside the storage container decreases. Conventionally, only less than 70% of the acetylene gas in a storage container has been taken out to prevent increases in the concentration of the residual solvent.

With use, the residual pressure inside the storage container decreases, which also results in a decrease in the flow rate of the acetylene gas taken out of the storage container per unit time. This means that not all the acetylene in the storage containers can be used, indicating a decrease in utilization rate.

[Prior Art Documents]

[Patent Document 1] US Patent No. 5702540
[Patent Document 2] US Patent Publication 2003/0168125
[Patent Document 3] International Patent Publication WO2008120160
[Patent Document 4] US Patent No. 8915992
[Patent Document 5] US Patent No. 8309473
[Patent Document 6] US Patent No. 8398747

[Summary of the Invention]

[Problems to be Solved by the Invention]

The present disclosure provides a gas refining system for removing trace impurities in gas. The present disclosure also provides an example of a system that is capable of appropriately removing trace amounts of residual solvent (solvent traces) in acetylene gas, and supplying acetylene (low-concentration residual solvent) that has been controlled in accordance with the specifications and conditions of a carburizing process.

The present invention also provides a system that, based on predicted results related to the use of the purifier, appropriately executes a regeneration step, and also allows gas refining system quality control and traceability to be executed.

[Means for Solving the Problems]

The gas refining system 1 for removing trace impurities in gas may comprise:

- a system unit 11 that comprises a gas source unit 111 comprising at least one storage container for storing a gas (such as acetylene gas) that is dissolved in an organic solvent, a purifier unit 113 for removing residual solvent (which is an organic solvent) from the gas, and a refining unit 112 for controlling the purification operations of the purifier unit 113;
- a monitoring unit 12 comprising a gas source monitor unit 121 for storing and monitoring data sent from the gas source unit 111, a purifier monitor unit 123 for storing and monitoring data sent from the purifier unit 113, and a refining monitor unit 122 for storing and monitoring data sent from the refining unit 112;
- an evaluation unit 13 for assessing the status of at least the purifier unit 113 (including purifier regeneration timing) by means of rule-based evaluation conditions and/or a machine learning model 131 based on one or more kinds of data from among system parameters 1100 (comprising various kinds of data that are used by the system unit 11), system monitoring data 1200 (comprising various types of monitor data used by the monitoring unit 12), and user parameters 1300 (comprising various type of parameters used by the gas refining system 1); and
- a control unit 15 comprising a purifier control unit 152 for controlling the purifier unit 113 based on data assessed by the evaluation unit 13.

The purifier unit 113 may comprise, for example, one or more purifiers (having adsorbent columns), regenerators for regenerating the purifiers, impurity concentration analysers, gas flow meters, memory devices, display devices, and various controllers.

The refining unit 112 monitors the concentration of impurities in the gas of the gas source unit 11 and/or the gas that has been purified by the purifier unit 113, and determines the proportions in which the gas from the gas source unit 11 (direct supply) and the purified gas are switched over or mixed (purification ratio X). Based on the determination of the need for purification, complete purification, or proportional purification, etc., acetylene gas is supplied to the point of demand point, with the residual solvent concentration (content) adjusted to no more than a given level. The refining unit 112 may also comprise, for example, monitoring devices such as impurity concentration analysers, gas pressure gauges, and thermometers; arithmetic processing units that process measurement data obtained from the various monitoring devices and determine the switch over timing and mixing proportions of purification ratio (X) for the purification treatment; and controllers for controlling the valves and mixers located in the piping.

The monitoring unit 12 may comprise a memory component for storing data sent from the gas source unit 111, the purifier unit 113, and the refining unit 112.

Evaluation" may include, for example, predictive evaluations at evaluation time points as well as future predictive evaluations.

The evaluation unit 13

has a memory component in which the at least one machine learning model 131 is stored, and

the machine learning model 131 may receive input of one or more kinds of data from among the system parameters 1100, the system monitoring data 1200, and the user parameters 1300, and may output an estimate of the status of at least the purifier unit 113.

The memory unit may store the system parameters 1100 sent from the monitoring unit 12, the system monitoring data 1200, and the user parameters 1300.

The evaluation unit 13 may comprise:

- a solvent evaluation subunit 132 for estimating the concentration of impurities in the gas (or the content of residual solvent in the gas) based on the estimation of the status of the purifier unit 113 output from the machine learning model 131;
- a purifier evaluation unit 134 for determining and/or assessing control information related to purification in the purifier unit 113 (such as the purifier replacement and regeneration time points) based on the user parameters 1300 and the concentration of impurities (or the content of residual solvent) in the gas estimated by the solvent evaluation subunit 132; and
- a refining evaluation unit 133 for determining and/or assessing the status of the purifier unit 113 (such as the determination that no purification is needed, the determination of proportional purification, or the determination of the mixing proportions such as purification ratio) based on the estimation of the status of the purifier unit 113 output from the machine learning model 131.

The refining control unit 151 may control the refining unit 112 (such as controlling when no purification is to be done or controlling the valves involved in proportional purification according to mixing proportions such as the purification ratio X) based on evaluations of the purifier unit 113 and/or gas source unit 111 obtained by the refining evaluation unit 133, and/or estimation of the status of the purifier unit 113 and/or gas source unit 111 output from the machine learning model 131.

The control unit 15 may have

- a purifier control unit 152 for controlling the purifier unit 113 (such as controlling purifier replacement and regeneration) based on the data determined by the purifier evaluation unit (134).

The machine learning model 131 may receive input of the system parameters 1100, the system monitoring data 1200, and the user parameters 1300, and may output estimates of the status of the purifier unit 113, the status of the gas source unit 111, and the control information of the refining unit 112.

The gas refining system 1 may have:

- a predictive evaluation memory component 14 for storing data about the status of the purifier unit 113, the status of the gas source unit 111, and the control information of the refining unit 112 that are obtained from the evaluation unit 13 and/or the machine learning model 131.

The gas refining system 1 may comprise:

- a system control/communication unit 16 that controls data about the status of the purifier unit 113, the status of the gas source unit 111, and the control information of the refining unit 112 that are sent from any of the evaluation unit 13, the predictive evaluation memory component 14, and the machine learning model 131, as well as the system parameters 1100 sent from the system unit 11.

The machine learning model 131 may be

- a machine learning model that has been trained using the following as training data: one or more kinds of data from among the system parameters 1100, the system monitoring data 1200, and the user parameters 1300; and the status data of the purifier unit 113.

The gas source pressure (or cylinder pressure), temperature, flow rate, quality of the carburized material (such as hardness or carburization depth), amount of soot generated, and maintenance frequency, for example, may be used as the training data.

The evaluation unit 13 may have:

- a memory component in which rule-based evaluation conditions are stored.

The evaluation unit 13 may check one or more of the system parameters 1100, the system monitoring data, and the user parameters 1300 against the rule-based evaluation conditions to assess the status of the purifier unit 113, assess the status of the gas source unit 111, and assess the control information of the refining unit 112.

The purifier unit 113 may have one or more purifiers 1131.

The purifier unit 113 may have one or more purifiers 1131 and a bypass pipe 1122 that does not pass through the purifier(s) 1131.

The one or more purifiers 1131 may be composed of in-series or in-parallel purification adsorbent columns or a condenser systems.

The purifier unit 113 may have: a main pipe L1 that is connected to the gas source unit 111;

- an upstream three-way valve 1123 upstream from the purifier 1131; a first branch pipe L11 that branches at the upstream three-way valve 1123; a second branch pipe L12 that branches at the upstream three-way valve 1123; and a downstream three-way valve 1124 where the first branch pipe L11 and the second branch pipe L12 downstream from the purifier 1131 merge.

The upstream three-way valve 1123 and the downstream three-way valve 1124 may be automatically driven valves or manual valves.

The upstream three-way valve 1123 and the downstream three-way valve 1224 may be controlled by control commands from the purifier control unit 152 and/or the purifier unit 113 or the refining unit 112. As an alternative to the three-way valve, a first valve may be provided upstream of the purifier 1121 in the first branch pipe L11, and a second valve may be provided in the second branch pipe L12.

Flow rate branch control means (branch valve, branch device) for controlling the flow rate of the first gas sent to the first branch pipe L11 and the flow rate of the second gas sent to the second branch pipe L12 may be used instead of the upstream three-way valve 1123.

A mixer 1124a or a buffer tank may be provided instead of the downstream three-way valve 1124, a first automatically driven valve may be provided in the first branch pipe L11 located upstream from the mixer 1124a or buffer tank and downstream from the purifier 1131, and a second automatically driven valve may be provided in the second branch pipe L12. A valve or an outlet valve may be provided on the outlet pipe of the mixer 1124a or the buffer tank. The configuration may be such that gas that has been purified in the purifier 1131 and gas that has not been purified are mixed in the mixer 1124a or the buffer tank, and are delivered downstream.

The first branch pipe L11 may be provided with a gas flow rate meter, gas pressure gauge, gas thermometer, gas concentration measuring device, and impurity concentration measuring device may be provided.

The second branch pipe L12, where no purifier 1131 has been provided, may be provided with a gas flow rate meter, gas pressure gauge, gas thermometer, gas concentration measuring device, and impurity concentration measuring device.

The pipe L1 downstream from the downstream three-way valve 11241 or mixer 1124a may be provided with a gas flow rate meter, gas pressure gauge, gas thermometer, gas concentration measuring device, and impurity concentration measuring device.

The purifier control unit 152 and/or the purifier unit 113 may determine the concentration (content) of impurities (residual solvent) in the feed gas from the storage container, as determined by an impurity concentration measuring device (or solvent analyser).

The refining control unit 151 and/or the refining unit 112 may, in response to downstream stage process (carburizing equipment) requirements,

- determine whether or not the gas should pass through the purifier 1131, and if so, how long the gas should pass through and the pressure range;
- determine whether or not the gas should pass through the bypass pipe 1122 (second branched pipe L2), and if so, how long the gas should pass through and the pressure range; and
- determine the proportions in which the purified gas that has passed through the purifier 1131 and the unpurified gas that has passed through the bypass pipe 1122 are mixed (proportions in which the gases are mixed per unit time: purification ratio X).

The refining evaluation unit 133 may determine the proportions of the purified gas (purification ratio X%) and the unpurified gas (1-X%).

The purifier control unit 152 may monitor and control changes in the status (such as in use, being regenerated, or on standby) or the maintenance status of the one or more purifiers 1131.

The purifier control unit 152 may determine and control which adsorbent column or adsorbent columns of the one or more purifiers 1131 should be used or regenerated. The purifier control unit 152 may monitor whether one or more purifiers 1131 are in use or are being regenerated (and on standby).

The purifier unit 113 and/or the purifier control unit 152 may have:

- a solvent accumulation calculation component for calculating the amount of impurities (solvent) that have been removed and accumulated in the purifier (1131);
- a purifier regeneration/replacement time calculation component for calculating when the purifier 1131 should be regenerated and/or replaced according to the amount of accumulated solvent obtained by the solvent accumulation calculation unit; and
- a regeneration control unit for controlling regeneration means according to the time for regenerating and/or replacing the purifier 1131 obtained by the purifier regeneration/replacement time calculation unit.

The regeneration means may have: a heating device for heating the adsorbent in the purifier; and an automatic gate valve provided in the pipe. The regeneration control unit may control turning the automatic gate valve on and off to switch the passage of gas to the purifier for regeneration or to the purifier for use. The regeneration control unit may control turning the heating device on to heat and regenerate the adsorbent in the purifier. The heating device may be an electric heating jacket provided on the exterior wall of a storage container (cylinder).

The regeneration means may be provided in the purifier unit 113 and may be provided as a regeneration unit.

The monitoring unit 12 may transmit various kind of data sensed by various monitor units 121, 122, and 123 to the evaluation unit 13 and the predictive evaluation memory component 14.

The various kinds of sensed data may be one or more of the kinds of data determined by various sensing devices (such as sensors) or computers, for example, that are provided in units 111, 112, and 113, such as: the temperature and residual pressure of structural elements of the gas refining system (1) or some of the storage containers (such as cylinders); quantitative analysis of gas sources; flow rate (such as cumulative volume and cumulative time) gas temperature, concentration (content) of impurities in gas, and gas purity of gas fed from one or more of the storage containers; flow rate (such as cumulative volume and cumulative time) of gas sent to the purifier; bypassed unpurified gas flow rate (such as cumulative volume and cumulative time); flow rate (such as cumulative volume and cumulative time) of gas sent to downstream stage processes; cumulative time of purifier use; time for which purifier is regenerated; and number of times purifier is regenerated.

The system control/communication unit 16 may acquire data about the system parameters 1100, system monitoring data 1200, user parameters 1300, gas source unit 111 status, refining unit 112 control information, and purifier unit 113 status, and transmit the data, as needed, to an external device or units (such as memory components), for example.

In an example of one embodiment, acetylene is used as the gas. The solvent vapour contained in the acetylene is removed or conditioned (removed to below a prescribed concentration value) before being fed to a process at the point of demand (carburizing equipment).

In an example of another embodiment, the gas refining system 1 may be used for other gases or gas mixtures, such as natural gas (NG) containing variable impurities such as carbon dioxide or water. For example, the purifier unit removes impurities in natural gas rather than residual solvent in acetylene gas. Other configurations may be adjusted, as appropriate, for different gases.

(Operational Advantages)

(1) The present invention allows acetylene gas of consistent purity to be extracted from a plurality of cylinders (storage containers) in which acetylene is stored.

(2) When the organic solvent is DMF, the present invention may be configured in such a way as to avoid extracting acetylene gas from cylinders in which the residual pressure has fallen below about 5 bar in order to prevent the solvent content of the acetylene gas from increasing to more than 500 ppm. The total number of cylinders that are used will thus be limited when the residual pressure has dropped. In the present disclosure, about 70 to 80% of the total stored acetylene can be supplied (only less than about 70% could conventionally be used).

(3) A purifier (solvent removal device) is provided, allowing the concentration (content) of residual solvent in the acetylene to be appropriately controlled by controlling, for example, whether gas is delivered, is not delivered, or is partially delivered to the purifier,

(4) A greater amount of acetylene gas is supplied from the storage containers, and the amount of residual acetylene in the storage container is lower than in the past, allowing a greater percentage of gas to be used (a lower percentage or residual gas).

(5) The flow rate of acetylene gas from a storage container can be maintained at a high level.

(6) Even when acetone is used as the organic solvent, the same quality (concentration of residual solvent) can be maintained as for DMF.

(7) The residual solvent in acetylene gas can be either completely or partially removed, and the acetylene purity can be increased to a prescribed allowable concentration level (the residual solvent concentration can be lowered to a certain level or lower).

(8) The size of the purifier equipment can be optimized.

(9) Renewable energy efficiency can be optimized.

(10) The evaluation unit enables evaluation of the timing at which gas is switched, such as when gas is not to be purified, is to be completely purified, or is to be proportionally purified.

[Brief Description of the Drawings]

[Figure 1] Function block diagram of gas refining system

[Figure 2] Diagram of structure of purifier unit and refining unit

[Figure 3] Example of a flow chart of purification treatment evaluation

[Figure 4] Diagram of the purification ratio X in cases A, B, and C

[Figure 5] Diagram of an example of piping configuration for direct flow and purification flow

[Figure 6] Examples of flow chart for switching complete purification on and off

[Figure 7] Example of a flow chart for switching point evaluation

[Figure 8] Example of a flow chart for re-evaluation

[Figure 9A] Example of a flow chart for purification ratio X evaluation and determination

[Figure 9B] Example of a flow chart for purification ratio X evaluation and determination

[Figure 9C] Example of a flow chart for purification ratio X evaluation and determination

[Figure 9D] Example of a flow chart for purification ratio X evaluation and determination

[Figure 10] Example of a flow chart for purifier status evaluation

[Modes for Carrying Out the Invention]

Several embodiments of the present invention are described below. The embodiment described below illustrates an example of the present invention. The present invention is in no way limited by the following embodiments, and also includes a number of variant modes which may be implemented without altering the essence of the present invention. It should be noted that not all of the configurations described below are essential configurations of the present invention.

(Embodiment 1)

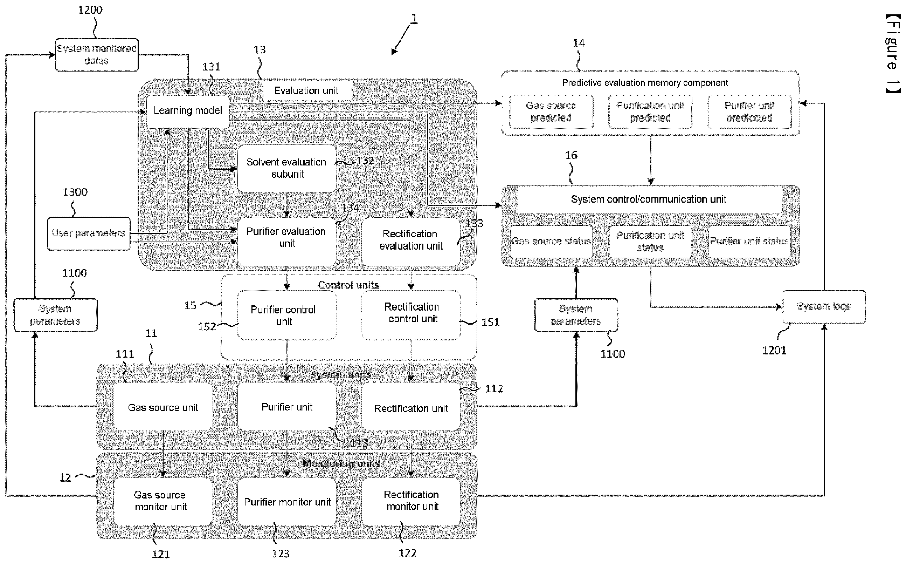

Figure 1 shows the various functions of the gas refining system 1 of Embodiment 1.

The gas refining system 1 comprises a system unit 11, a monitoring unit 12, an evaluation unit 13, a predictive evaluation memory component 14, a control unit 15, and a system control/communication unit 16.

The machine learning model 131 may be a machine learning model (software) that has been trained using training data, and may comprise a dedicated AI chip (including firmware installation).

The system unit 11 has: a gas source unit 111 comprising one or more storage containers for storing acetylene gas; a purifier unit 113 for removing residual solvent in the acetylene gas; and a refining unit 112 for controlling the purification operations in the purifier unit 113.

The gas source unit 111 may have, for example: one or more storage containers (such as cylinders) for storing the gas, piping for sending gas from the storage containers to downstream stage equipment (such as carburizing equipment), valves (such as gate valves and flow rate control valves) that are provided in the storage containers or piping, gas flow rate meters for measuring the gas flow rate that are provided in the storage containers or piping, gas pressure gauges for measuring gas pressure that are provided in the storage containers or piping, gas thermometers for measuring gas temperature that are provided in the storage containers or piping, gas concentration measuring devices for measuring the main gas concentration that are provided in the storage containers or piping, and impurity concentration measuring devices (solvent analysers) for measuring the concentration of impurities (residual solvent) in gas that are provided in the storage containers or piping.

The gas source unit 111 may determine the content of impurities (residual solvent) in the feed gas, as determined by a solvent vapour analyser.

The gas source monitor unit 121 may have a memory component where data measured by the various measurement devices is acquired (received) and is stored with their respective associated measurement times and respective instrument identification information.

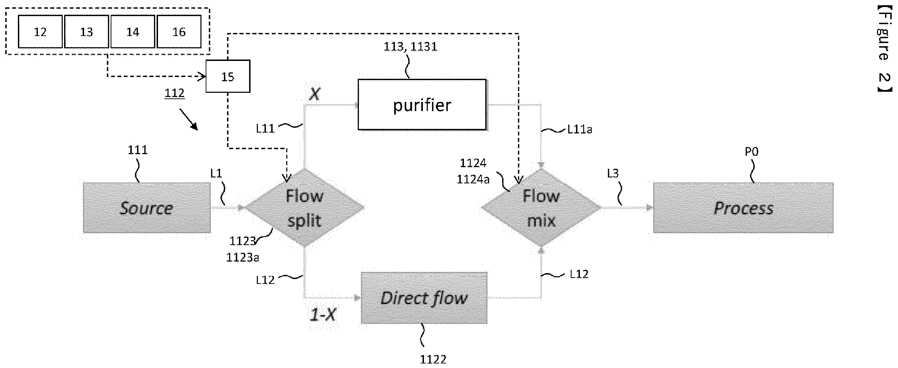

Figure 2 is a diagram of the structure of the purifier unit 113 and the refining unit 112. Figure 2 illustrates, for example, a gas source unit 111, a purifier unit 113, a refining unit 112, a flow split 1123 (also referred to as "distribution means"), merging means 1124, and a control unit 15.

The gas in Embodiment 1 is acetylene gas. The acetylene is dissolved in an organic solvent (DMF) and is then impregnated into a porous material to be stored in a storage container. The purifier unit 113 comprises one or more purifiers 1131. The purifiers 1131 may comprise in-series or in-parallel purifying adsorbent columns or a condenser system.

The flow split 1123 is means for distributing the acetylene gas supplied from the gas source unit 111 (delivered entirely in any direction, distributed at a prescribed flow rate). The flow split 1123 may be composed of a three-way valve 1123a, a damper mechanism, a control valve, a flow rate control valve, a proportional flow rate control valve, or a solenoid valve, for example.

The merging means 1124 allows acetylene gas that has been processed and purified in the purifier unit 113 (purifiers 1131) and acetylene gas that is supplied, without having undergone purification treatment, from the gas source unit 111. The merging means 1124 allows purified acetylene gas alone to be delivered to a downstream stage, allows unpurified acetylene gas alone to be delivered to a downstream stage, and allows both to be mixed in a prescribed mixing ratio (purification ratio) before being delivered to a downstream stage. The merging means 1124 allows unpurified acetylene gas and purified acetylene gas (that has undergone purification treatment in the purifier unit 113) (purifiers 1131) to be mixed in a prescribed purification ratio (X = 0 to 1). The merging means 1124 may be composed of a downstream three-way valve 1124a, and may comprise a T-shaped pipe and a gate valve or a flow rate control valve.

The merging means 1124 may comprise a mixer 1124a or a buffer tank. The mixer 1124a or the buffer tank may be configured so that the gas that has been purified in the purifiers 1131 and the gas that has not been purified are mixed, allowing gas having a purification ratio (X) to be delivered downstream.

The area where the gas is mixed in the flow mix means 1124 may be composed of a mixing chamber or a buffer chamber. In the case of a buffer chamber, the purified acetylene gas and the directly delivered unpurified acetylene gas may be introduced at the same time at a controlled flow rate, or may be sequentially introduced into the buffer chamber based on set volume fractions. The area where the gas is mixed can be equipped with one or more sampling ports, and the solvent in the acetylene gas and/or other impurities may be measured by an analyser.

The first pipe L1 is a pipe for delivering unpurified acetylene gas from the gas source unit 111 to the flow split 1123. The first branch pipe L11 is a pipe for delivering unpurified acetylene gas from the flow split 1123 to the purifier unit 113. The second branch pipe L11a is a pipe for delivering purified acetylene gas from the purifier unit 113 to the merging means 1124. The bypass pipe L12 is a pipe for delivering unpurified acetylene gas from the flow split 1123 to the merging means 1124. The lead-out pipe L3 is a pipe for delivering acetylene gas from the merging means 1124 to the point of demand PO at a downstream stage.

The storage containers and/or each of the above pipes may include a valve (for example, a gate valve, a flow rate control valve), a flow rate meter for measuring the gas flow rate, a gas pressure gauge for measuring the gas pressure, a gas thermometer for measuring the gas temperature, a gas concentration measuring device for measuring the gas concentration, an impurity concentration measuring device (solvent trace analyser) for measuring the concentration of impurities (solvent) in the gas, and so on. The data measured in each of these may, as system parameters 1100 or system monitoring data 1200, be transmitted with their respective associated measurement times and respective instrument identification information to the monitoring unit 12 and/or evaluation unit 13, and may be stored in a memory component (not shown).

The system parameters 1100 may comprise, for example, the initial pressure in the cylinder, the cylinder residual pressure measured in real time during use, the gas temperature, and the gas flow rate (total extraction flow rate).

The system monitoring data 1200 may comprise, for example, various kinds of data that affect the concentration of residual solvent (such as acetone or DMF) in acetylene gas, including the residual pressure in the cylinder, gas temperature, gas flow rate, and residual solvent concentration (solvent content).

The user parameters 1300 may comprise, for example, the upper limit value of the concentration of impurities (residual solvent content) that is needed to ensure a favourable carburizing process.

(Monitoring Unit)

The monitoring unit 12 comprises a gas source monitor unit 121, a purifier monitor unit 123, and a refining monitor unit 122.

Gas source monitor unit 121 stores and monitors data sent from the gas source unit 111. Purifier monitor unit 123 stores and monitors data sent from the purifier unit 113. The refining monitor unit 122 stores and monitors data sent from the refining unit 112.

The gas source monitor unit 121 comprises, for example: gas temperature sensors for measuring the temperature of the gas in the gas storage containers (cylinders) or the gas in the piping connected to the gas storage containers; gas pressure sensors for measuring the pressure of the gas; and gas flow rate sensors for measuring the flow rate of the gas flowing through the piping connected to the gas storage containers.

The purifier monitoring unit 122 comprises, for example: gas temperature sensors for measuring the temperature of the gas flow in the piping leading to the purifier 1131 or the gas flow in the pipe passing through the purifier; gas pressure sensors for measuring the pressure of the gas; and gas flow sensors for measuring the flow rate of the gas flow. The purifier monitor unit 123 comprises: for example impurity concentration measuring devices for measuring the concentration of impurities of the gas in the piping leading to the purifiers 1131 or the gas in the piping passing through the purifier.

The refining monitor unit 122 comprises, for example, a calculation component for calculating the cumulative gas flow rate of the gas flow introduced into the purifier 1131 while in use, and calculating the cumulative gas flow rate of the bypassed gas.

The purifier monitor unit 123 comprises, for example: temperature sensors for measuring the temperature of the heating device (an example of regenerating means) provided in the purifiers 1131; and temperature sensors for measuring the temperature of the adsorbent in the purifiers. The purifier monitor unit 123 also comprises, for example, a calculation component for calculating the cumulative number of times that the purifiers are regenerated and for how long they are regenerated.

(Evaluation Unit)

The evaluation unit 13 comprises a machine learning model 131, a solvent evaluation subunit 132, a purifier evaluation unit 134, and a refining evaluation unit 133.

The machine learning model 131 receives the input of one or more kinds of data from among the system parameters 1100, the system monitoring data 1200, and the user parameters 1300, and outputs an estimate of the status of at least the purifier unit 113.

The solvent evaluation subunit 132 estimates the content of residual solvent in the gas based on the estimation of the status of the purifier unit 113 output from the machine learning model 131.

The solvent evaluation subunit 132 estimates the concentration of impurities in the gas flow based on the estimation of the status of the purifier unit 113 output from the at least one machine learning model 131. The solvent evaluation subunit 132 directly or indirectly estimates the amount of solvent (or impurities) in the gas flow. Impurities present in trace amounts in the gas flow comprise, for example, solvent (such as acetone or DMF) that is in the acetylene derived from the acetylene-based storage source (such as the cylinder).

The concentration of impurities may be directly estimated using, for example, a method of measurement method such as an infrared (FTIR or NDIR) detector or photo-ionization detector, or an analyser such as a gas chromatograph. The measurement may be in-line measurement, or impurity-specific sensors may be used.

The concentration of impurities may be indirectly estimated by the machine learning model 131 via input of the gas source temperature ($T_{source}$), internal temperature ($T_{room}$), external temperature ($T_{ext}$), gas flow rate Q, residual pressure of the source gas ($P_{res}$), and system data 1200. Additional information related to the gas source may also be input, such as the filling pressure (initial pressure, $P_{res}$, 0), nature of the solvent, and type of storage (such as Noral or Noral 2).

The machine learning model 131 also receives input of the system parameters 1100, the system monitoring data 1200, and the user parameters 1300, and outputs estimates of the status of the purifier unit 113, the status of the gas source unit 111, and the control information of the refining unit 112.

The purifier evaluation unit 134 determines control information related to purification in the purifier unit 113 based on the user parameters 1300 and the content of residual solvent in the gas estimated by the solvent evaluation subunit 132. The control information comprises, for example, the time points at which the purifier is replaced or regenerated.

The refining evaluation unit 133 determines the status of the purifier unit 113 based on the estimation of the status of the purifier unit 113 output from the machine learning model 131. The status of the purifier unit includes, for example, the determination that no gas is to be purified, the determination of proportional purification, and the determination of the proportions in which the gas is mixed, such as the purification ratio.

(Predictive Evaluation)

The predictive evaluation memory component 14 stores data about the status of the purifier unit 113, the status of the gas source unit 111, and the control information of the refining unit 112 that are obtained from the evaluation unit 13 and/or the machine learning model 131. The predictive evaluation storage unit 14 stores the system log 1201 output from the monitoring unit 12.

(System Control/Communication Unit)

The system control/communication unit 16 controls data about the status of the purifier unit 113, the status of the gas source unit 111, and the control information of the refining unit 112 that are sent from any of the evaluation unit 13, the predictive evaluation memory component 14, and the machine learning model 131, as well as the system parameters 1100 sent from the system unit 11.

The system control/communication unit 16 controls data about the system parameters 1100, gas source unit 111 status, refining unit 112 control information, and purifier unit 113 status obtained from the system unit 11, and transmits the information, as needed, to an external units or external device.

The system control/communication unit 16 may comprise, for example, communication means for communicating various kinds of data to one or more users. The communication means may be composed of wireless or wired communication means, and communications may be accomplished in real time with the use of an encrypted or unencrypted data transfer system, such as over an Internet connection, a local network, or IoT, or a delayed method may be used.

(Control Unit)

The control unit 15 comprises a purifier control unit 152 and a refining control unit 151. The purifier control unit 152 controls the purifier unit 113 based on data determined by the purifier evaluation unit 134. The time points at which the purifiers are replaced or regenerated are controlled, for example.

The refining control unit 151 controls the purification treatment based on the evaluation of the purifier unit 113 obtained by the refining evaluation unit 133, and or the estimation of the status of the purifier unit 113 output from the machine learning model 131.

The unit controls, for example, when gas is not purified and controls valves involved in proportional purification according to the proportion in which the gas is mixed, such as the purification ratio X.

(Purification Treatment)

The refining control unit 151 controls the flow rate control valves or gate valves provided in pipes L1, L11, L12, and L3, as well as the flow split 1123 and the merging means 1124, based on the purification ratio (X). The refining control unit 151 controls the branching means or the valves in accordance with the purification ratio X. The amount of gas delivered to the purifier 1131 and the amount of gas delivered to the bypass pipe L12 are controlled. Gas flow rate meters, gas pressure gauges, gas thermometers, gas concentration measuring devices, and impurity concentration measuring devices may be provided in the pipes. The measured data is delivered with their respective associated measurement times and respective instrument identification information to the monitoring unit 12, and is stored in a memory device.

(Method of Determining the Limiting Residual Pressure)

The evaluation unit 13, the control unit 15, and/or the system unit 11 determine the limit residual pressure of the storage containers according to specific conditions of use (flow rate, temperature). An allowable residual solvent (impurity) content in the acetylene gas is first determined in accordance with the conditions of use, and the residual pressure at which the quality of the target carburizing process cannot be obtained is determined.

When the internal pressure measured in a storage container that is in use drops below the limiting residual pressure, use is switched to another storage container. This switching is referred to as the switching point (point of limiting use). This point of limiting use can be used to determine when the purification treatment by the purifier unit 113 starts and ends. The switching point can also be determined based on data such as the data measured by the various measurement devices (system monitoring data 1200, system parameters 1100), or the residual pressure or temperature in the storage containers. The switching point is calculated based on information that can be measured, such as the residual pressure and the temperature of the storage containers, the solvent content in the acetylene gas, the cumulative supply time, the total amount of acetylene gas supplied, and the flow rate of the acetylene. The purification system can be optimized (such as number of purifiers and size of adsorbent columns) by determining the switching point.

The concentration of the acetylene gas (residual solvent concentration) extracted from the container may be measured in advance, as correlated with the residual pressure and temperature, an approximation function may be determined based on the data, and the approximation function (may be a linear function) may be stored in the memory component. This approximation function can be used to calculate the acetylene gas concentration (residual solvent concentration) based on the measurement values for the residual pressure and the temperature.

Replacement of the storage container, use of purifiers, use of the bypass pipe, use of the mixing means, and so on, may be determined in accordance with the acetylene concentration and flow rate required at the point of demand PO.

The purifiers also do not always need to be used, and the system can be smaller compared with systems in which purifiers are constantly used. Smaller purifiers make it possible to reduce the energy needed for regenerating the purifiers.

Three types of purification treatment are performed in accordance with the switching point.

(1) Proportional Purification

The amount of gas to be purified and the amount of gas not to be purified are changed and mixed in real time or at specific time intervals (one minute units, 10 minute units, etc.) in accordance with the change in the calculated purification ratio (X). Purification of the acetylene gas is varied with the fixed maximum residual solvent content in the acetylene gas as the upper limit.

(2) Fixed Ratio Purification

When it is judged that purification is necessary, the purification treatment is performed in accordance with a predetermined fixed purification ratio.

(3) Complete Purification (or Non-purification)

When it is judged that purification is necessary, 100% of the acetylene gas is purified (the gas is delivered to the purifier unit, without delivering gas to the bypass pipe). When it is judged that purification is not necessary, 100% of the acetylene gas is delivered to a downstream stage (the gas is delivered to the bypass pipe, without delivering gas to the purifier).

To determine the switching point, the relationship between the residual pressure of the storage container during use and the concentration of the acetylene gas (concentration of the solvent) to be extracted can be specified by a predetermined function, based on storage container initial values (volume, filling pressure, and solvent amount) and the acetylene solubility. This function can be used to determine the ideal acetylene supply that can be used in the downstream stage carburizing process.

(a) A generally used working pressure limit can be determined from the storage container residual pressure.
(b) Depending on the target final use pressure (residual pressure), and depending on the conditions of use (flow rate and temperature), it is possible to achieve an increase of 10% to 60% of the acetylene gas that can be used. For example, in the case of a DMF cylinder (long term use from 5 bar to 2 bar), the amount of acetylene that can be used is increased from about 20% to 30%, and in the case of an acetylene cylinder (long term use from 6 bar to 2 bar), the amount of acetylene that can be used is increased from about 35% to 40%.

The target purification ratio (X) is determined based on the estimated solvent content ($\eta_{direct}$) and user requirements (target solvent content $\eta_{target}$). This can be determined by the evaluation unit 13 and control unit 15 using various methods.

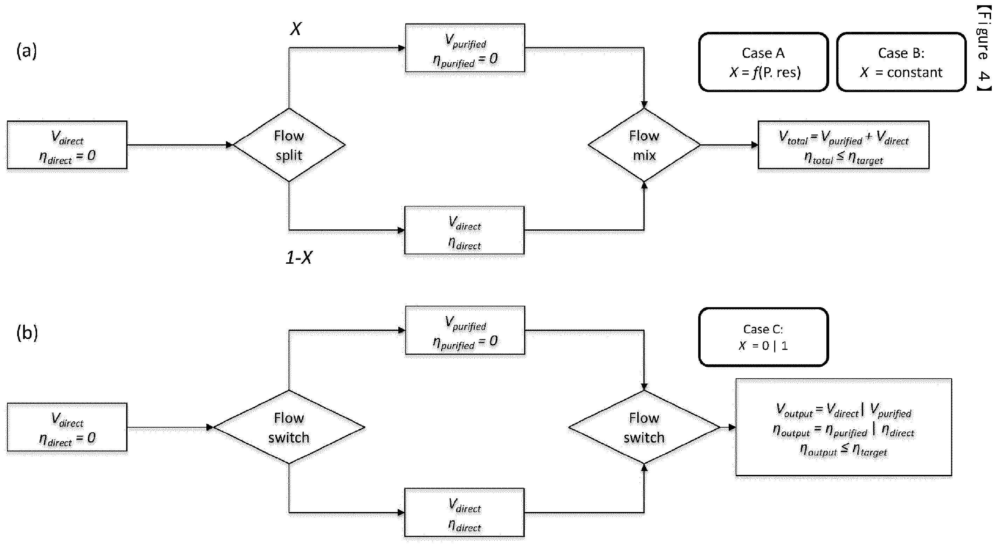

Figure 4 illustrates two approaches. Proportional purification (cases A and B) and complete purification (case C) are illustrated. The main difference between these two approaches is that, in proportional purification, a portion of the acetylene gas from the storage container is purified (purification ratio X), and in contrast, in complete purification, all the acetylene gas from the storage container is purified after switching.

(1) Case A: After the switching point is reached, purification is performed to a purification ratio (X) that is varied in accordance with the evaluation results (Figure 4(a))
(2) Case B: After the switching point is reached, purification is performed to a fixed purification ratio (X) (Figure 4(a))
(3) Case C: After the switching point is reached, all the acetylene gas is purified (Figure 4(b))

Figure 4(a) illustrates proportional purification with a purification ratio (X) in which the acetylene gas is distributed so that only a portion of the acetylene gas is purified. Figure 4(b) illustrates complete purification in which the acetylene gas from the storage container (cylinder) is completely purified by the purifier unit 113.

The fixed purification ratio (X) may be set to a fixed ratio in accordance with the requirements at the final point of demand PO. The variable purification ratio (X) is determined as the target value of the maximum fixed content of the residual solvent in the acetylene gas.

Proportional Purification

The evaluation unit 13 and the control unit 15 determine the amount (proportion) of the gas to be purified in the purifier unit 113. Next, the purified gas is mixed with the unpurified gas that has not undergone a purification treatment to produce a gas mixture. The content of solvent (impurity) in the gas mixture corresponds to processing requirements at a downstream stage.

The advantage of the gas refining system 1 comprising the purifier unit 113 and refining unit 112 is that the purifier unit can be smaller compared with a purifier system in which the total amount of acetylene gas supplied to the process is purified at a downstream stage.

In this embodiment, the purification ratio X of the purified gas may be a fixed value. The purification ratio may be determined by re-evaluation. The variable purification ratio may be determined by dynamic evaluation.

In another embodiment, the purification ratio X or whether or not complete purification is needed may be determined based on fixed values such as the target final residual pressure (before storage container replacement). In such cases, the purification ratio X is determined as a single value by a predictive method. A fixed purification ratio X may furthermore be estimated based on the number of times used in a specific range, the gas volume, or the residual pressure (which can be considered to be intermediate between Cases A and B).

The proportion of gas to be purified (purification ratio X) is determined based on the ideally evaluated solvent (impurity) content of the storage container ($\eta_{source}$), target solvent (impurity) content ($\eta_{target}$), and the solvent (impurity) content in the purified acetylene gas after purification ($\eta_{purified}$).

The solvent (impurity) content of the gas mixture after purification can be determined, or the solvent (impurity) content as a result of switching to complete purification is determined.

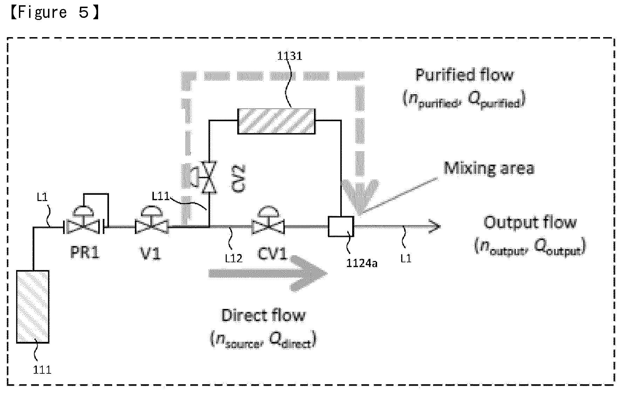

Figure 5 illustrates an example of purification treatment with the purifier unit 113 and the refining unit 112. This system can produce acetylene gas of a predetermined purity by purifying some or all of the acetylene from the acetylene gas source unit 111 using the purifier unit 113 and mixing the purified gas with unpurified acetylene gas.

The relationship between each solvent concentration and the purification ratio X is shown by Formula (1).

$$(\text{Formula 1})\eta_{output} = (1\text{-X}).\eta_{source} + \text{X}.\eta_{purified}$$

The relationships for the total gas flow rate $Q_{total}$ (= $Q_{source}$ = $Q_{output}$) are shown in Formula (2), Formula (3), and Formula (4). Here, the state before the switching point corresponds to X = 0.

$$(\text{Formula 2})Q_{total} = Q_{purified} + Q_{direct}$$

$$(\text{Formula 3})Q_{purified} = \text{X}.Q_{total}$$

$$(\text{Formula 4})Q_{direct} = (1\text{-X}).Q_{total}$$

Flow rate of acetylene gas from the storage container: $Q_{source}$ (= $Q_{total}$)
Solvent content in the acetylene gas from the storage container: $\eta_{source}$
Flow rate of acetylene gas subjected to the purification treatment in the purifier: $Q_{purified}$ Solvent content (concentration) in the acetylene gas after the purification treatment in the purifier: $\eta_{purified}$
Flow rate of acetylene gas that has bypassed the purifier: $Q_{direct}$
Solvent content in the acetylene gas that has bypassed the purifier: $\eta_{direct}$
Flow rate of acetylene gas output from purifier unit 113 and refining unit 112: $Q_{output}$ Solvent content in the acetylene gas output from purifier unit 113 and refining unit 112: $\eta_{output}$
Target solvent content from the user's requirements: $\eta_{target}$
Pressure regulating valve with pressure gauge: PR1
Upstream flow rate control valve: V1
Flow rate control valve provided on the purifier pipeline L11: CV2
Flow rate control valve provided on the bypass pipeline L12: CV1
The branching means 1123 can function with CV1 and CV2.

The residual solvent (and impurities) in the acetylene gas extracted from the storage containers can be diluted by the purifier unit 113 and the refining unit 112. (1) The purifier unit 113 may comprise one or more columns filled with adsorbent or a solvent condenser. The purifier unit 113 can generate an acetylene gas flow of higher purity (from which at least 50% of the solvent vapour has been removed, for example) from the acetylene gas delivered from the storage container with.

(2) The purified acetylene gas and the unpurified acetylene gas can be mixed or the conditions of use can be controlled.

The purifier unit 113 and the refining unit 112 allow most of the acetylene gas in the storage container to be used while also lowering the amount of residual solvent in the acetylene gas supplied to the process at a downstream stage.

For example, standard DMF solvent storage containers having the prescribed (or allowable) DMF content of 500 ppm (target solvent content $\eta_{target}$) conventionally could no longer be used when the residual pressure reaches 5 bar. Assuming this is an appropriate shut down of a storage container while in use, only about 60% to 70% of the total amount of the acetylene gas in the storage container could be used. In the gas refining system 1, on the other hand, the storage container can be used to a residual pressure of 2 bar without exceeding the target solvent content $\eta$target, and 70% to 80% of the total amount of the gas in the storage container can be used. Therefore it is possible to extend the use of the storage containers (cylinders, etc.) on site, and reduce the frequency of replacement and replenishment of storage containers.

The evaluation unit 13 and control unit 15 can determine the status and how much should be purified.

Purity of the acetylene in the supplied gas < target purity of the acetylene in the purified gas Solvent content in the supplied gas > solvent content in the purified gas (gas mixture) When the quality of acetylene gas drops below the standard value and it is not possible to switch to another storage container, the evaluation unit 13 and the control unit 15 may control the gate valves in order to completely stop the flow from the gas source unit 11 (storage container).

The evaluation unit 13 and control unit 15 may estimate the total amount of acetylene gas used based on (i) the conditions of use (residual pressure in the storage container when starting, and residual pressure during use) and (ii) an integral of the flow rate of the acetylene gas delivered to the process at the point of demand.

The evaluation unit 13 and control unit 15 may acquire or determine information about the current percentage of gas that has been used in the storage container (cylinder), the predicted time until the switching point as well as the time remaining until the storage container (cylinder) is replaced (usable time, replacement time), in accordance with any of the mean values based on the pre-registered carburizing processing time, use thereof, or recorded data. The evaluation unit 13 and control unit 15 may output an alarm to the user when a problem occurs due to cooling of the storage container associated with extraction of the acetylene gas. An alarm may be issued to the user under when there are excessive changes in the conditions of use (flow rate problem, temperature, residual pressure too low), etc. Alerts can also be issued when a predetermined percentage of the gas in the storage container has been used.

The evaluation unit 13 and control unit 15 may have one or more processors that may read various kinds of data and control commands from a memory component to execute the processes required for the various types of control.

The evaluation unit 13 and control unit 15 may exchange various kinds of data and command signals with an external device. The external device may be, for example, the gas source unit 111, flow split 1123, purifier unit 113, merging means 1124, memory device, cloud server, cloud storage, control device at the point of demand PO, or user controlled device.

Examples of various kinds of data include the above measurement data, determined data, purification ratio X, switching point (point of limiting use), estimated time until a storage container is replaced, residual pressure, various kinds of processing data, and control command data.

This data may be used to improve management of various aspects, such as inventory and quality control.

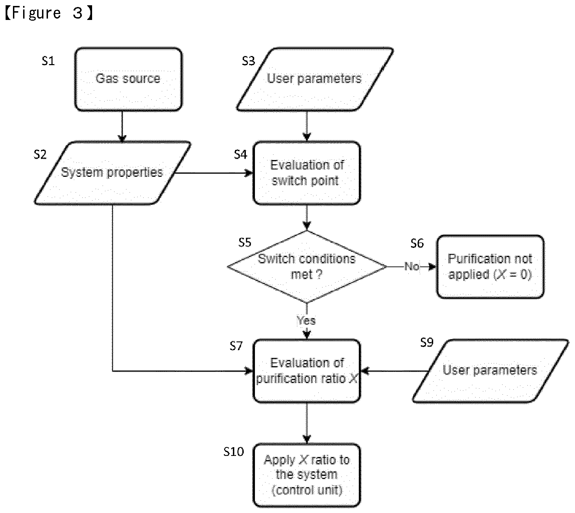

Figure 3 shows an example of a flow chart for purification treatment evaluation.

Gas is supplied from the gas source unit 111 (S1), and the monitoring unit 12 senses various kinds of system monitor data 1200 (S2). The various types of system monitor data 1200 that have been sensed are transmitted to the evaluation unit 13.

Various user parameters 1300 are input to the evaluation unit 13 (S3). The refining evaluation unit 133 determines the switching point (SP) for determining whether purification is needed (S4). It is determined whether the switching point conditions have been met (S5). If the conditions are not met, no purification treatment is executed (S6). If the conditions are met, the purification ratio (X) is determined (S7) based on the input user parameters (S9). For example, the target purification ratio (X) is determined based on a user parameter (impurity level tolerance), estimated impurity level (actually measured value), and system status. The control unit 15 delivers gas to the purifier 1131 and carries out purification based on this target purification ratio (X) (S10).

The evaluation unit 13 and control unit 15 may control and monitor the entire system. Table 1 shows case examples of purification treatments.

Residual pressure of the storage pressure during use: $P_{residual}$

Switching point: Sp = limiting residual pressure in normal use. In the present disclosure, the amount of residual solvent is controlled via purification at residual pressures lower than that.

[Table 1]

| | | Purification ratio X[(a)] Re-evaluation rate | Conditions |
|---|---|---|---|
| Case A | | From no purification to purification (X=0 to 0.9) Proportional purification | $P_{residual}$>Sp |

(continued)

| | | Purification ratio X[a] Re-evaluation rate | Conditions |
|---|---|---|---|
| Case B-1 | | Purification (fixed, with optional range of X=0.1 to 0.9) <br> Fixed proportional purification <br> Purification carried out within set time | When abnormalities are detected[b] |
| Case B-2 | | Purification (fixed, with optional range of X=0.1 to 0.9) <br> Fixed proportional purification <br> Purification carried out until measured residual pressure returns to normal range | When abnormalities are detected[b] |
| Case C | | Complete purification 100% (X=1) <br> Not delivered to bypass piping <br> Purification carried out until Presidudal returns to final | $P_{residual} < Sp$ |
| (a): Purification ratio X: The amount of purified acetylene gas purified by the purifier unit/total amount of unpurified acetylene gas extracted from the storage container <br> (b): Temperature, residual pressure, or other measurement parameters from the control unit that involve significant changes from normal values or estimated values. | | | |

(Embodiments of Purification)

The evaluation unit 13 and control unit 15 may determine the limiting residual pressure before replacement of a storage container and the range of pressure margin (offset value) higher than the limiting residual pressure corresponding to the limiting purity level (lower limit concentration, or the target solvent content $\eta_{target}$) determined in advance for the carburizing process at the demand point.

Purification treatments comprise: proportional purification (Case A); fixed ratio purification (Case B); and complete purification (Case C). In proportional purification, only a portion of the acetylene gas is purified (purification ratio X). In complete purification, all the acetylene gas is purified. The purification treatment can be set based on the switching point (including offset). Fixed ratio purification (case B) is when purification is performed at a fixed set purification ratio for a certain period of time and when purification is performed at a fixed set purification ratio until the residual pressure returns to normal range.

Proportional Purification

Proportional purification is when some of the acetylene gas extracted from a storage container is purified.

The evaluation unit 13 and control unit 15 determine the amount of acetylene gas to be purified in the purifier 1131. The purified acetylene gas is mixed with the unpurified acetylene gas in the merging means 1124. The residual solvent content (solvent concentration) in the acetylene gas is thus reduced. The use of a partial purification treatment allows the purifier unit to be smaller compared with other systems in which purification is constantly taking place.

The amount of purified acetylene gas delivered from the purifier unit 113 (purification ratio X) and the amount of unpurified acetylene gas directly delivered to the carburizing process at the point of demand PO (1-X) are controlled to provide at total flow rate ($V_{total}$) that is suitable for one or more carburizing processes. Note that the amount of purified acetylene gas (purification ratio X) and the amount of unpurified acetylene gas (1-X) are mixed in the merging means 1124 (such as a mixer 1124a) and delivered to the point of demand PO.

Solvent content in the purified acetylene gas: $\eta_{purified}$
Amount of purified acetylene gas: $V_{purified}$
Amount of unpurified acetylene gas directly delivered: $V_{direct}$

(Formula 11)

$$V_{total} = V_{purified} + V_{direct}$$

(Formula 12)

$$V_{purified} = X.V_{total}$$

(Formula 13)

$$V_{direct} = (1\text{-}X).V_{total}$$

(Formula 14)

$$\eta_{total} = (1\text{-}X).\eta_{total} + X.\eta_{purified}$$

The solvent content in the acetylene gas extracted from the standard storage container (acetylene cylinder) ($\eta_{direct}$) can be appropriately expressed as a function of the residual pressure in the storage container. For example, the function can be a logistics function, or a linear function in the region where the residual pressure is restricted by a fixed temperature and a fixed gas flow rate.

In other words, the solvent content in the acetylene gas extracted from the storage container ($\eta_{direct}$) can be obtained based on data such as the acetylene flow rate, the residual pressure, the temperature, and so on.

(Conditional Purification)

In conditional purification (Figure 4(b)), the acetylene gas is either completely purified, subject to conditions, or it is not purified.

The conditions are determined by the control unit based on process requirements, gas flow rate, storage container temperature, residual pressure, and other measurement values. The timing for switching the flow switch for executing either complete purification or no purification is determined as a specific value of the residual pressure (for example, 5 bar). The purification treatment is carried out with system constituted as illustrated in Figure 5. The acetylene gas delivered from the system to the carburizing process is either completely purified or is not purified.

Before switching: The solvent content in the acetylene gas output is the same as $\eta_{direct}$, $V_{direct}$. After switching: The solvent content in the acetylene gas output is $\eta_{purified}$, $V_{purified} \fallingdotseq V_{direct}$. The control unit 15 controls and monitors the valves. Examples of such valves include solenoid valves, pneumatic valves, or pneumatic or solenoid three-way valves.

(Complete Purification)

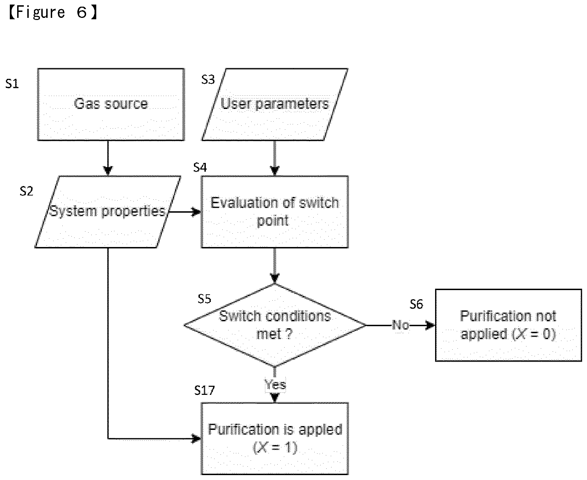

Figure 6 shows a flow chart for refining system evaluation during complete purification (Case C).

Gas is supplied from the gas source unit 111 (S1), and the monitor unit 12 senses various kinds of system monitor data 1200 (S2). The various types of system monitor data 1200 that have been sensed are transmitted to the evaluation unit 13.

Various user parameters 1300 are input to the evaluation unit 13 (S3). The refining evaluation unit 133 determines the timing at which the flow is switched for determining whether purification is needed (S4). It is determined whether conditions for the timing at which the flow is switched have been met (S5). If the conditions are not met, no purification treatment is executed (S6). If the conditions are met, the purification rate (X=100) is determined (S17).

The process to be followed, the gas source, the system parameters, and the user parameters are used as a function of the evaluation of the timing for switching. The evaluation can be determined based on estimates of gas impurity concentrations, other system parameters, or a combination of both.

(Refining Evaluation Model)

The evaluation unit 13 evaluates whether the switching point (SP) conditions having been met. The switching point (SP) can be represented by a Boolean value, for example. If the Boolean value is TRUE, the gas flow must be purified; if the Boolean value is FALSE, the gas flow does not need to be purified.

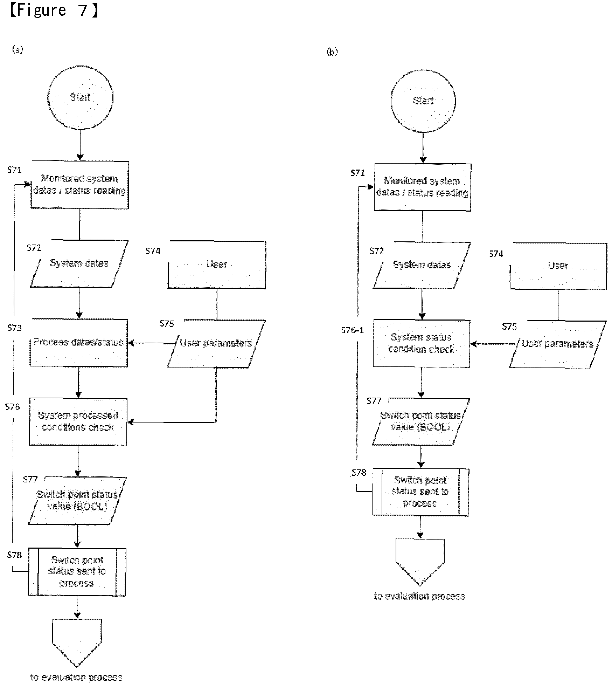

Figure 7 shows examples of flow charts for switching point evaluation. In Figure 7(a), an evaluation is made (S76) as to whether the system parameters 1100 acquired from the system unit 11 and the system monitor data 1200 acquired from the monitoring unit 12 (S71, S72, S73) meet the input user parameters 1300 (S74, S75). In this way, the status value (Boolean value) of the switching point (SP) is determined (S77). The status value (Boolean value) of the switching point (SP) is transmitted to the evaluation process (S78).

In Figure 7(b), an evaluation is made by directly comparing the system parameters 1100 acquired from the system unit 11 and the system monitor data 1200 acquired from the monitoring unit 12 (S71, S72) with the user parameters 1300 that have been input (S74, S75) to check the condition of the system status (S76-1). In this way, the status value (Boolean value) of the switching point (SP) is determined (S77). The status value (Boolean value) of the switching point (SP) is transmitted to the evaluation process (S78).

The switching point (SP (Eval)) is distinguished from the switching point based on the purification time determined by the refining control unit ($S_P$ (CU)) 151.

(Evaluation)

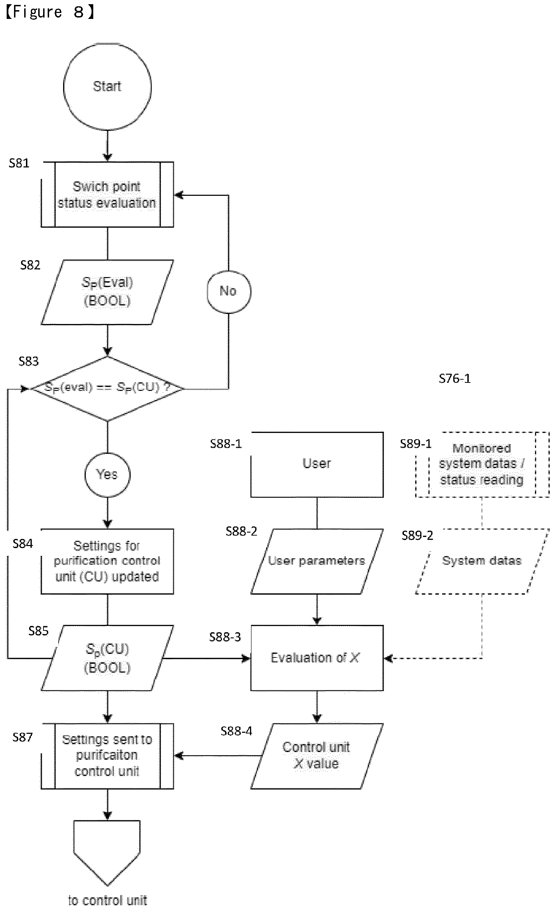

The evaluation process is dynamically repeated. Figure 8 is a flow chart for re-evaluation. The function and goal of the re-evaluation is to evaluate changes in the system and to ascertain whether the control unit 15 settings need to be re-evaluated. The control unit 15 settings correspond to current setting values or various kinds of data.

Evaluation of the switching point status is started (S81). The status value (Boolean value) of the switching point (Sp(eval)) determined in Figure 7 is acquired (S82). Next, it is determined whether the value of the switching point (Sp(Eval)) matches the value of the control unit 15 ($S_P$(CU)) (S83). If the values do not match, the process returns to step S81 and the step is repeated.

If the values do match, the settings of the refining control unit ($S_P$ (CU)) are updated (S84). The refining control unit ($S_P$ (CU)) value is set as the switching point (Sp(Eval)) (S85). The set value is transmitted to the refining control unit.

The user parameters (S88-1, S88-2) input by the user, the monitored system data (S89-1, S89-2), and the purifier control unit (SP (CU)) values are compared, and the purification ratio (X) is evaluated (S88-3). The purification ratio X is transmitted to the control unit 15 (S88-4).

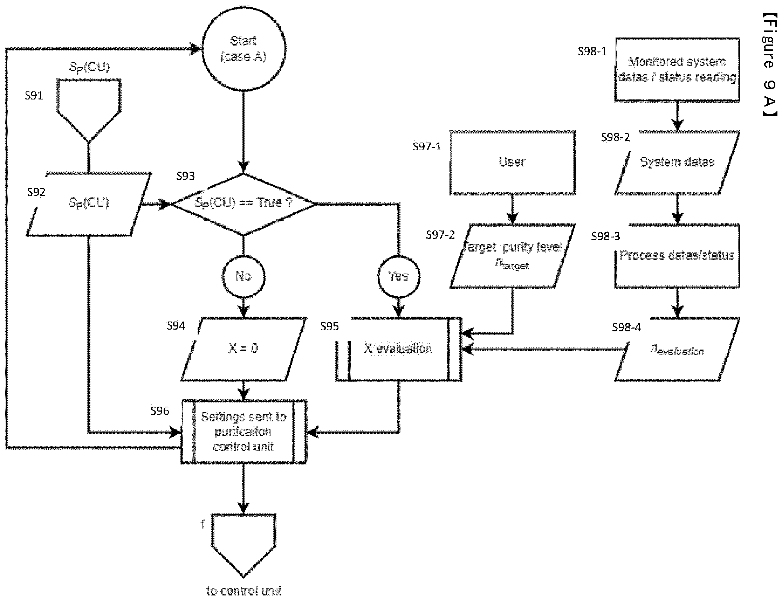

Figures 9A through 9D show processes for evaluating and determining the purification ratio X.

(Figure 9A: Case A)

Evaluation of purification ratio X based on estimated value $\eta_{evaluation}$ of the impurity concentration at the t-nth time

The refining control unit (SP (CU)) value determines whether purification is necessary (True) (S93). If it is determined that no purification is needed, the purification ratio X is set to 0 (S94) and the set value is transmitted to the refining control unit 151 (S96).

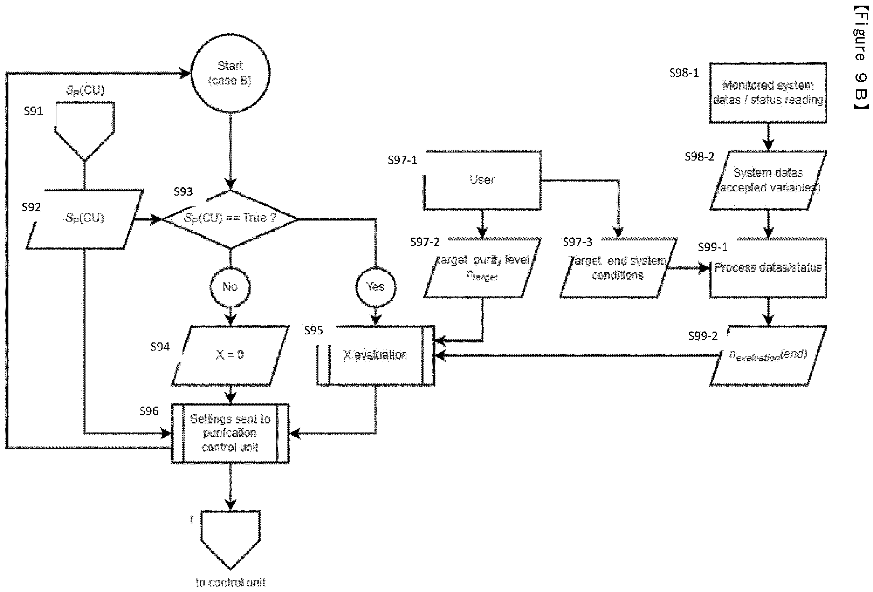

If it is determined that purification is needed, the purification ratio X is evaluated (S95). The target impurity concentration $\eta_{target}$ requested by the user is transmitted to the evaluation process (S95) (S97-1, S97-2). The system monitoring data 1200 and the system data 1100 are used to process the estimated value $\eta_{evaluation}$ of the impurity concentration (S98-1, S98-2, S98-3, S98-4), and the estimated value $\eta_{evaluation}$ is transmitted to the evaluation process (S95). The evaluated purification ratio X is transmitted to the refining control unit 151 (S96). (Figure 9B: Case B) Evaluation of purification ratio X based on estimated value $\eta_{evaluation}$ of the impurity under conditions at the point of demand

The process differs from that in Case A in that the system status at the point of demand point (S97-3) is transmitted to the process data (S99-1), the estimated value $\eta_{evaluation}$ (end) of the impurity concentration is processed (S99-2), and the estimated value $\eta_{evaluation}$ (end) is transmitted to the evaluation process (S95) and evaluated.

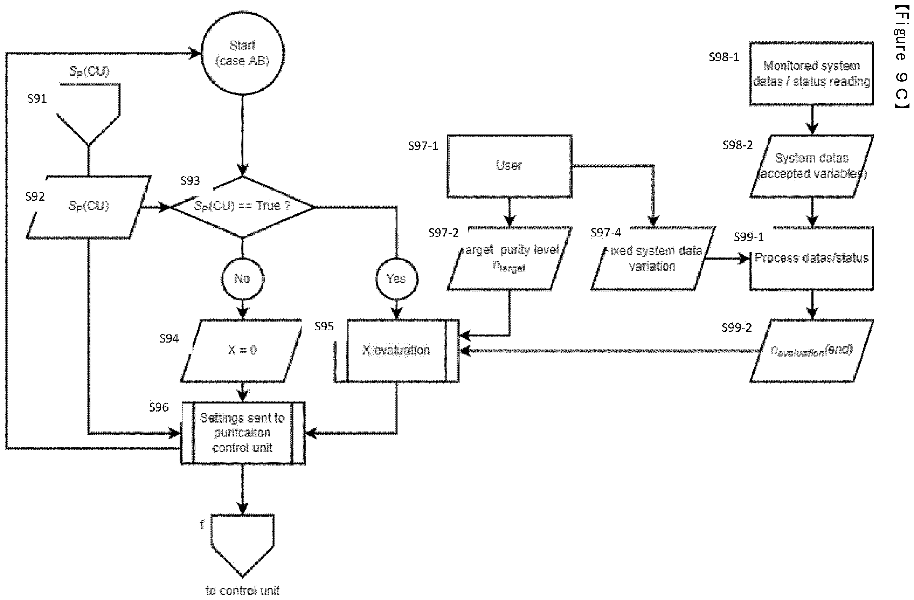

(Figure 9C: Cases B-1 and B-2)

Evaluation of purification ratio X based on estimated value $\eta_{evaluation}$ of the impurity concentration when conditions are changed

The process differs from that in Case A and Case B in that fixed system data (S97-4) is transmitted to the process data (S99-1), the estimated value $\eta_{evaluation}$ (end) of the impurity concentration is processed (S99-2), and the estimated value $\eta_{evaluation}$ (end) ) is transmitted to the evaluation process (S95) and evaluated.

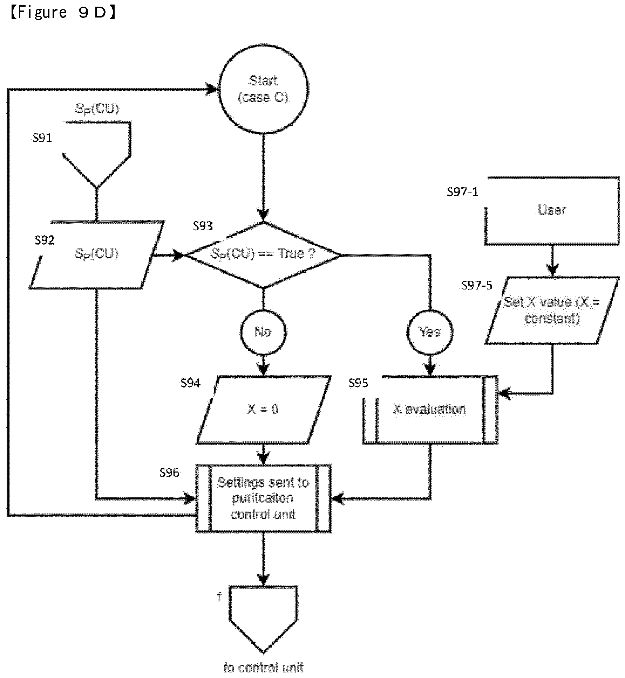

(Figure 9D: Case C)

Setting the Purification ratio X as a fixed value according to the switching point The process differs from that in case A in that the fixed value X (S97-5) is transmitted to the evaluation process (S95), and the purification ratio X is evaluated.

The size (number, dimensions) of a plurality of purifiers (such as adsorbent columns) can be determined by determining the Purification ratio X. With this refining system 1: (i) gas sources that exceed current recommended limits can be used; and (ii) impurity-related problems, such as certain safety risks, maintenance costs, and process quality assurance, can be proactively prevented from exceeding predetermined tolerances;

The purifier footprint can also be smaller compared to standard systems in which purifiers (such as adsorbent columns) are continuously used. Systems that are more efficient regenerated (lower regeneration energy) based on the overall output of the system can also be developed. The energy waste and carbon footprint (CFP) of the system are also immediately lowered.

(Purifier Unit)

The purifier unit 113 comprises one or more purifiers 1131. The purifier 1131 may include a first refining device for removing minute quantities of solvent as a liquid, a removal device or purifier for removing minute quantities of solvent such as a mechanical trap or a cold trap (second refining device), or a third refining device for removing other minute impurities (such as sulphur or phosphine compounds, and so on).

The purifier 1131 may comprise one or more columns in which a filling material is housed. Examples of the filling material may include one or more of zeolites, exchange zeolites, activated carbon, processed (or impregnated) activated carbon, metal-organic frameworks (MOF), covalent organic frameworks (COF), polymer, and silica materials. The filling material may be in the form of, for example, film, monolith, pellets, beads, blocks, or powder.

The purifier 1131 may comprise columns arranged in series, columns arranged in parallel, or both of these column arrangements.

The purifier control unit 152 may comprise: an accumulation calculation component for calculating the amount of residual solvent (also referred to as "impurities") that have been removed (purified) by the purifier unit 113 and have accumulated in the purifier (such as the adsorbent column); a regeneration/replacement time calculation component for calculating when the purifiers 1131 of the purifier unit 113 should be regenerated or replaced according to the amount of accumulated residual solvent calculated by the accumulation calculation unit; and a regeneration control unit for controlling regeneration means (not shown) according to the time for regenerating and/or replacing the purifiers 1131 calculated by the purifier regeneration/replacement time calculation unit.

The regeneration means may include a heating device for heating the adsorbent in the purifier; regeneration gas supply means for supplying the regeneration gas (that is used to regenerate adsorbent) via a pipe; and an automatic gate valve that is provided in the pipe.

The regeneration control unit may control when the automatic gate valve is turned on and off to switch the passage of gas through the purifier for regeneration or the purifier for use. The regeneration control unit may control when the heating device is turned on to heat and regenerate the adsorbent in the purifier. The heating device may be an electric heating jacket provided on the exterior of the wall of a storage container. The regeneration control unit may control commands to the regeneration gas supply means, the timing at which the regeneration gas is supplied to the piping, and the time for which the gas is supplied.

When the content of impurities changes, the purification ratio X is not simply correlated to the when purification is or is not carried out and the time for which purification is carried out. The evaluation unit 13 estimates the amount of impurities that have been removed or trapped by the purifier. The status of the purifier (column) during and after the refining process is determined and predicted based on system parameters such as the adsorbent removal capacity of the purifier (such as column), the filling amount, and the purification treatment status (percentage of gas used).

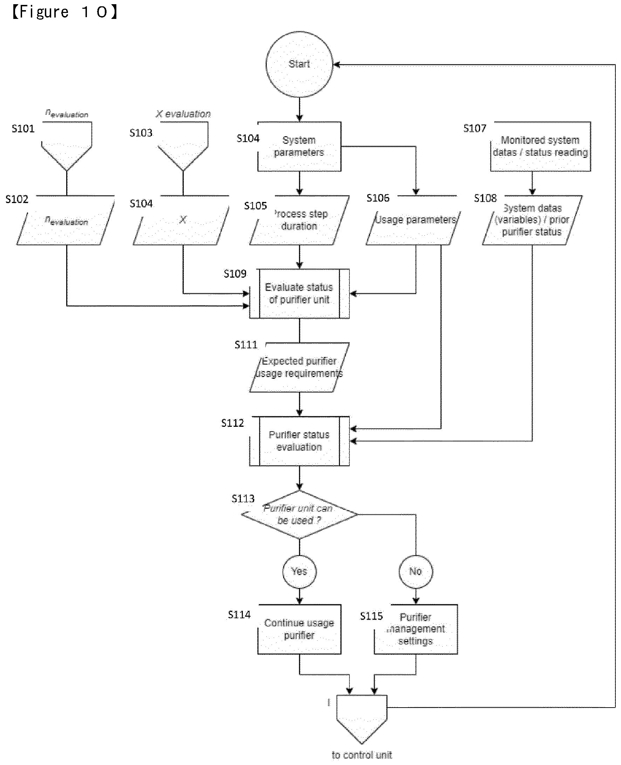

Figure 10 is a flow chart for evaluating the purifier status.

The estimated value of the impurity concentration $\eta_{evaluation}$ (S101, S102), the evaluated purification ratio (S103, S104), the system parameters (S104), the duration of the step (S105), and the usage parameters (S106) are input to evaluate the purification unit (S109). Next, a comparison is made with the expected period of time used (purification capacity) for the purification treatment (S111). Monitor system data (S107) is acquired, and the system data as well as the previous purifier status are transmitted to the purifier status evaluation process (S112) (S108). The current status of the purifier is evaluated based on the requested time for use, usage parameters, system data, and the last prior status of the purifier (S112). Based on the evaluation results, a determination is made as to whether the purification treatment can be continued or whether the regeneration process should be performed (or the unit replaced) (S113). If it is determined that the purification treatment can be continued, the use of the purifier is continued (S114). If it is determined that the purification process cannot be continued, the purifier regeneration process or replacement information is set (S115).

(Machine Learning Model for Impurity Evaluation)

The evaluation of impurity concentrations in gas sources depends on the nature of the gas, the type of gas source, and the impurities under consideration, and can also depend on the parameters of use.

Direct evaluation is preferred when impurities are a parameter that is difficult to predict, such as water vapour in air or water vapour in methane. If the impurities are a parameter that can be determined, both indirect and direct evaluation with reasonable accuracy based on system parameters may be considered, and may be compared where applicable.

When the acetylene contains acetone, for example, the acetone content is estimated at a fixed flow rate as a function of temperature and residual pressure. The solvent content in the acetylene can therefore be estimated based on these two parameters.

A database that includes the gas source cylinder temperature, room temperature, and amount of acetylene used will be useful for producing a more sophisticated and more accurate predictive model for the concentration of impurities in acetylene.

One or more impurity concentration evaluation models determine the concentration of impurities using the cylinder temperature and residual pressure as well as other parameters of. One or more impurity concentration evaluation models can also be used to predict drops in cylinder temperature during use or variations in other parameters during use. The output data generated by the impurity concentration evaluation model can be used for subsequent model development and the accuracy of system parameter variation.

For example, the concentration of impurities in acetylene may be estimated based on only the cylinder temperature and residual pressure. The estimate may then be validated via comparison with a specific measurement method to confirm. A combination of parameter estimates can be used to further enhance the accuracy of the impurity concentration assessment model as well as the capacity for the system to be used predictively. This may be used to optimize the system that is being used.

Thus, in one embodiment, it will be possible to provide one or more impurity concentration evaluation models for estimating the concentration of impurities that can dynamically evolve based on available data. This dynamic model is ideally disposed and used in processes, which can be in a factory or a data centre, or both, in order to optimize them to develop a single kind of data. Some or all of the impurity concentration evaluation models may be centralized to allow variations that are significantly greater than expected to be detected and communicated to users.

(Evaluation of Concentration of Impurities to be Eliminated)

The evaluation unit 13 evaluates the concentration of impurities in gas that is going to be purified. The evaluation unit 13 includes at least one structural element capable of directly or indirectly evaluating the solvent or impurities present in a flow of gas. Methods of measurement involving infrared (FTIR or NDIR) detectors or photo-ionization detectors, or analysers such as a gas chromatographs, are examples that may be used for direct measurement. The measurement may be in-line measurement, or impurity-specific sensors may be used.

The predicted concentration of impurities is determined (predicted) based on the machine learningmodel 131. The machine learning model 131 is determined and set based on variable parameters that are available for the evaluation in one or more units under consideration. System monitoring data 1200, system parameters 1100, and/or user parameters 1300 can be used as such parameters. The data used are estimated based on (i) prior variations observed in the units (such as changes in residual pressure or cylinder temperature in relation to the volume of acetylene used) and/or (ii) selected parameter variations. The predicted concentration of impurities therefore reflects the influence of parameter variations in an estimated manner. The results obtained from data input can furthermore be compared with one or more database values to facilitate their validation. Such comparisons can be used to allow the predictive model 131 to be dynamically updated, as needed.

The concentration of impurities is then evaluated using the machine learning model 131, and the results are compared. The estimated concentration of impurities is then compared with (i) the previous values of the units under consideration and (ii) the predicted concentration impurities for outputting estimated concentrations of impurities related to accuracy and reliability. These impurity estimates are recorded in the unit databases and are used in one or more machine learning models 131. The machine learning model 131 is used to provide equivalent values for impurities based on previous data and the machine learning model 131 and based on an evaluation of input (parameter) evolution.

[Key]

1: Gas refining system; 11 System unit; 12: Monitoring unit 13: Evaluation unit; 15: Control unit; 16: System control/communication unit

**Claims**

1. A gas refining system comprising: a system unit 11 comprising a gas source unit 111 that comprises at least one storage container for storing a gas that is dissolved in an organic solvent, a purifier unit 113 for removing residual solvent, which is an organic solvent, from the gas, and a refining unit 112 for controlling the purification operations of the purifier unit 113;

   - a monitoring unit 12 comprising a gas source monitor unit 121 for storing and monitoring data sent from the gas source unit 111, a purifier monitor unit 123 for storing and monitoring data sent from the purifier unit 113, and a refining monitor unit 122 for storing and monitoring data sent from the refining unit 112;
   - an evaluation unit 13 for assessing the status of at least the purifier unit 113 by means of rule-based evaluation conditions and/or a machine learning model 131 based on one or more kinds of data from among system parameters 1100, which comprise various kinds of data that are used by the system unit 11, system monitoring data 1200, which comprise various types of monitor data used by the monitoring unit 12, and user parameters 1300, which comprise various type of parameters used by the gas refining system 1; and
- a control unit 15 comprising a purifier control unit 152 for controlling the purifier unit 113 based on data assessed by the evaluation unit 13.

2. The gas refining system according to Claim 1, wherein the evaluation unit 13 has a memory component in which the at least one machine learning model 131 is stored, and
the machine learning model 131 receives one or more kinds of input data from among the system parameters (1100), the system monitoring data (1200), and the user parameters (1300), and outputs an estimation of the status of at least the purifier unit 113.

3. The gas refining system according to Claim 2, wherein the evaluation unit 13 comprises:

- a solvent evaluation subunit 132 for estimating the concentration of impurities in the gas based on the estimation of the status of the purifier unit 113 output from the machine learning model 131;
- a purifier evaluation unit 134 for determining and/or assessing control information related to purification in the purifier unit 113 based on the user parameters 1300 and the concentration of impurities in the gas estimated by the solvent evaluation subunit 132; and
- a refining evaluation unit 133 for determining and/or assessing the status of the purifier unit 113 based on the estimation of the status of the purifier unit 113 output from the machine learning model 131.

4. The gas refining system according to Claim 3, wherein a refining control unit 151 controls the refining unit 112 based on: the evaluation of the purifier unit 113 and/or gas source unit 111 obtained by the refining evaluation unit 133; and or the estimation of the status of the purifier unit 113 and/or gas source unit 111 output from the machine learning model 131.

5. The gas refining system according to Claim 1, wherein the evaluation unit 13 has a memory component in which rule-based evaluation conditions are stored, and
the evaluation unit 13 checks one or more of the system parameters 1100, the system monitoring data, and the user parameters 1300 against the rule-based evaluation conditions to assess the status of the purifier unit 113, assess the status of the gas source unit 111, and assess the control information of the refining unit 112.

【Figure 1】

【Figure 2】

【Figure 3】

S1 Gas source

S3 User parameters

S2 System properties

S4 Evaluation of switch point

S5 Switch conditions met ?

No

S6 Purification not applied ($X = 0$)

Yes

S7 Evaluation of purification ratio $X$

S9 User parameters

S10 Apply $X$ ratio to the system (control unit)

【Figure 4】

(a)

| Case A | Case B: |
|---|---|
| X = f(P. res) | X = constant |

$V_{direct} = 0$
$\eta_{direct} = 0$

Flow split

$X$

$V_{purified}$
$\eta_{purified} = 0$

$1-X$

$V_{direct}$
$\eta_{direct}$

Flow mix

$V_{total} = V_{purified} + V_{direct}$
$\eta_{total} \leq \eta_{target}$

(b)

Case C:
X = 0 | 1

$V_{direct} = 0$
$\eta_{direct} = 0$

Flow switch

$V_{purified}$
$\eta_{purified} = 0$

$V_{direct}$
$\eta_{direct}$

Flow switch

$V_{output} = V_{direct} \mid V_{purified}$
$\eta_{output} = \eta_{purified} \mid \eta_{direct}$
$\eta_{output} \leq \eta_{target}$

【Figure 5】

【Figure 6】

【Figure 7】

(a)

```
        ┌─────────┐
        │  Start  │
        └────┬────┘
             │
             ▼
S71   ┌──────────────────┐
      │ Monitored system │
      │datas/status reading│
      └────────┬─────────┘
               │
S72   ┌────────┴────────┐        S74  ┌──────────────┐
      │  System datas   /             │     User     │
      └────────┬────────┘             └──────┬───────┘
               │                             │
S73   ┌────────┴────────┐    S75   ┌─────────┴──────────┐
      │Process datas/status│◄───────/  User parameters  /
      └────────┬────────┘          └─────────┬──────────┘
               │                             │
S76   ┌────────┴────────┐                    │
      │ System processed │◄───────────────────┘
      │ conditions check │
      └────────┬────────┘
               │
S77   ┌────────┴────────┐
      / Switch point status/
      /  value (BOOL)     /
      └────────┬────────┘
               │
S78   ┌────────┴────────┐
      │   Switch point   │
      │  status sent to  │
      │     process      │
      └────────┬────────┘
               │
               ▼
         to evaluation process
```

(b)

```
        ┌─────────┐
        │  Start  │
        └────┬────┘
             │
             ▼
S71   ┌──────────────────┐
      │ Monitored system │
      │datas/status reading│
      └────────┬─────────┘
               │
S72   ┌────────┴────────┐        S74  ┌──────────────┐
      │  System datas   /             │     User     │
      └────────┬────────┘             └──────┬───────┘
               │                             │
S76-1 ┌────────┴────────┐    S75   ┌─────────┴──────────┐
      │  System status  │◄───────/   User parameters   /
      │ condition check │          └────────────────────┘
      └────────┬────────┘
               │
S77   ┌────────┴────────┐
      / Switch point status/
      /  value (BOOL)     /
      └────────┬────────┘
               │
S78   ┌────────┴────────┐
      │   Switch point   │
      │  status sent to  │
      │     process      │
      └────────┬────────┘
               │
               ▼
         to evaluation process
```

【Figure 8】

S81 — Swich point status evaluation

S82 — $S_P(Eval)$ (BOOL)

No

S83 — $S_P(eval) == S_P(CU)$ ?

Yes

S84 — Settings for purification control unit (CU) updated

S85 — $S_p(CU)$ (BOOL)

S76-1

S88-1 — User

S89-1 — Monitored system datas / status reading

S88-2 — User parameters

S89-2 — System datas

S88-3 — Evaluation of $X$

S87 — Settings sent to purifcaiton control unit

S88-4 — Control unit $X$ value

to control unit

[Figure 9 A]

EP 4 570 353 A1

[Figure 9 B]

Start
(case B)

$S_P$(CU)

S91

S92

$S_P$(CU)

S93

$S_P$(CU) == True ?

No

Yes

S94

X = 0

S95

X evaluation

S96

Settings sent to
purifcaiton
control unit

f

to control unit

S97-1

User

S97-2

Target purity level
$n_{target}$

S97-3

Target end system
conditions

S98-1

Monitored system
datas / status reading

S98-2

System datas
accepted variables)

S99-1

Process datas/status

S99-2

$n_{evaluation}$(end)

[Figure 9 C]

EP 4 570 353 A1

Start
(case AB)

$S_P(CU)$

S91

S92  $S_P(CU)$

S93  $S_P(CU)$ == True ?

No

Yes

S94  X = 0

S95  X evaluation

S96  Settings sent to purifcaiton control unit

S97-1  User

S97-2  target purity level $n_{target}$

S97-4  Fixed system data variation

S98-1  Monitored system datas / status reading

S98-2  System datas (accepted variables)

S99-1  Process datas/status

S99-2  $n_{evaluation}(end)$

f

to control unit

30

【Figure 9D】

【Figure 10】

S101 $n_{evaluation}$

S103 X evaluation

S104 System parameters

S107 Monitored system datas / status reading

S102 $n_{evaluation}$

S104 X

S105 Process step duration

S106 Usage parameters

S108 System datas (variables) / prior purifier status

S109 Evaluate status of purifier unit

S111 Expected purifier usage requirements

S112 Purifier status evaluation

S113 Purifier unit can be used ?

Yes

No

S114 Continue usage purifier

S115 Purifier management settings

to control unit

Europäisches
Patentamt

European
Patent Office

Office européen
des brevets

# EUROPEAN SEARCH REPORT

**Application Number**

EP 24 21 3868

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 116 399 937 A (L AIR LIQUIDE SA POUR L ETUDE ET L EXPLOITATION DES PROCEDES GEORGES C) 7 July 2023 (2023-07-07) <br> * figure 1 * <br> * claims 1,7 * <br> * page 9, second last paragraph of the machine translation * | 1-5 | INV. <br> B01D53/00 <br> B01D53/04 <br> B01D53/30 <br> C07C7/12 <br> C07C11/24 |
| X | US 2023/257878 A1 (CHANDRASEKHARAN RAMESH [US] ET AL) 17 August 2023 (2023-08-17) <br> * figures 2A-C * <br> * paragraphs [0081] - [0092], [0149] - [0151] * | 1-5 | |
| A | US 2011/311724 A1 (JENSEN BEN POUL [GB] ET AL) 22 December 2011 (2011-12-22) <br> * paragraph [0033] * | 1-5 | |
| A | WO 2008/120160 A1 (AIR LIQUIDE [FR]; LETESSIER OLIVIER [US] ET AL.) 9 October 2008 (2008-10-09) <br> * figure 1 * | 1-5 | **TECHNICAL FIELDS SEARCHED (IPC)** <br><br> B01D <br> C07C |

The present search report has been drawn up for all claims

| Place of search | Date of completion of the search | Examiner |
|---|---|---|
| Munich | 1 May 2025 | Pöhlmann, Robert |

EPO FORM 1503 03.82 (P04C01)

## ANNEX TO THE EUROPEAN SEARCH REPORT
## ON EUROPEAN PATENT APPLICATION NO.

EP 24 21 3868

This annex lists the patent family members relating to the patent documents cited in the above-mentioned European search report.
The members are as contained in the European Patent Office EDP file on
The European Patent Office is in no way liable for these particulars which are merely given for the purpose of information.

01-05-2025

| Patent document cited in search report | Publication date | Patent family member(s) | Publication date |
|---|---|---|---|
| CN 116399937 A | 07-07-2023 | CN 116399937 A<br>EP 4390605 A1<br>JP 2024084701 A<br>US 2024191839 A1 | 07-07-2023<br>26-06-2024<br>25-06-2024<br>13-06-2024 |
| US 2023257878 A1 | 17-08-2023 | CN 115917039 A<br>JP 2023535548 A<br>KR 20230045590 A<br>TW 202223147 A<br>US 2023257878 A1<br>WO 2022026271 A1 | 04-04-2023<br>18-08-2023<br>04-04-2023<br>16-06-2022<br>17-08-2023<br>03-02-2022 |
| US 2011311724 A1 | 22-12-2011 | CN 102292287 A<br>EP 2382157 A2<br>GB 2467320 A<br>JP 2012516278 A<br>KR 20110128179 A<br>SG 173082 A1<br>US 2011311724 A1<br>WO 2010086600 A2 | 21-12-2011<br>02-11-2011<br>04-08-2010<br>19-07-2012<br>28-11-2011<br>29-08-2011<br>22-12-2011<br>05-08-2010 |
| WO 2008120160 A1 | 09-10-2008 | US 2008242912 A1<br>WO 2008120160 A1 | 02-10-2008<br>09-10-2008 |

EPO FORM P0459

For more details about this annex : see Official Journal of the European Patent Office, No. 12/82

**REFERENCES CITED IN THE DESCRIPTION**

**Patent documents cited in the description**

- US 5702540 A
- US 20030168125 A
- WO 2008120160 A
- US 8915992 B
- US 8309473 B
- US 8398747 B